# EUROPEAN PATENT APPLICATION

(11) **EP 2 314 271 A2**
(43) Date of publication of application: **27.04.2011**
(21) Application number: 10186929.5
(22) Date of filing: 08.10.2010
(51) Int. Cl.: A61K 8/06, A61K 8/29, A61K 8/41, A61K 8/894, A61Q 17/04, B01F 17/18

(54) **Stable water-in-oil emulsion system**

(30) Priority: 21.10.2009 CN 200910205260
(71) Applicant: Evonik Goldschmidt GmbH, 45127 Essen (DE)
(72) Inventor: Zhang, Haizhou, 201108, Shanghai (CN); Dai, Jingya, 201108 Shanghai, (CN); Zou, Jiali, 201108, Shanghai (CN); Gao, Zhiheng, 201108, Shanghai (CN)

(57) **Abstract**

Summary

The water-in-oil emulsion system of the present invention comprises a water phase, an oil phase, a water-in-oil emulsifier of 1-5 wt% on the basis of the total amount of the system, a solid powder of 0.1-20 wt% on the basis of the total amount of the system and a cationic surfactant of 0.01-2 wt% on the basis of the total amount of the system, in which the cationic surfactant is the cationic surfactant of a quaternary ammonium salt, represented by the general formula R₁ R₂R₃R₄N⁺X⁻, in which R₁, R₂, R₃ and R₄ are each independently selected from a C₁-C₂₈ alkyl group or a C₁-C₂₈ alkyl group substituted by a C₁-C₄ acyl group, a C₂-C₄ acyloxy group, a hydroxy group or a C₂-C₄ alkenyl group, and X⁻ is any suitable anion.

## Description

### Technical Field

The present invention relates to a stable water-in-oil emulsion system.

### Background Art

Water-in-oil powder-containing systems of low viscosity currently available on the market are sunscreening emulsions (shake-shake formulations), low-viscosity foundation emulsions, etc. Common problems generally existing in those products are that the emulsion systems are unstable and easily result in phase separation or powder sedimentation during their long-term use.

US patent no. US7,138,128 B2 discloses a composition using cation-containing polymers to improve the stability of a W/O system with a low viscosity and a high water content. The inventor demonstrated by tests that the stability of the system still could not meet the requirements of commercial products.

Therefore, a water-in-oil emulsion system with good stability even under low viscosity is currently expected by the market.

### Object of the invention

An object of the present invention is to provide a stable water-in-oil emulsion system with low viscosity, which contains a cationic surfactant. The water-in-oil emulsion system of the present invention realizes the stabilization of a water-in-oil emulsion system under the conditions of low viscosity, a relatively low amount of emulsifier and emulsified particles of 1-10µm. The water-in-oil emulsion system of the present invention produces no apparent phenomena of delamination, sedimentation or oil-water separation when centrifugally treated at 3000rpm for 30min.

### Contents of the invention:

The water-in-oil emulsion system of the present invention comprises a water phase, an oil phase, a water-in-oil emulsifier of 1-5 wt% on the basis of the total amount of the system, a solid powder of 0.1-20 wt%, preferably a hydrophobic solid powder, on the basis of the total amount of the system and a cationic surfactant of 0.01-2 wt% on the basis of the total amount of the system, in which the cationic surfactant is the cationic surfactant of a quaternary ammonium salt, represented by the general formula R₁R₂R₃R₄N⁺X⁻, in which R₁, R₂, R₃ and R₄ are each independently selected from a C₁-C₂₈ alkyl group or a C₁-C₂₈ alkyl group substituted by a C₁-C₄ acyl group, a C₂-C₄ acyloxy group, a hydroxy group or a C₂-C₄ alkenyl group, and X⁻ is any suitable anion anion such as Cl⁻, Br or CH₃SO₃-.

In the water-in-oil emulsion system of the present invention, the contents of the oil phase and the water phase, without including the above-mentioned solid powder, the cationic surfactant and the water-in-oil emulsifier, are 5-65 wt% for the oil phase, preferably 10-45 wt%, and 30-90 wt% for the water phase, preferably 45-85 wt%.

In the water-in-oil emulsion system of the present invention, the oil phase is one normally contained in a water-in-oil emulsion, without any other specific limitation on the selection of each of the components in the oil phase, and for which all components allowable for use in cosmetics can be used. They can be at least one oil/fat selected from vegetable oils, mineral oils, silicon oils and synthesized oils that are traditionally used, and can also be various waxes that are traditionally used.

The oil/fat of silicon oil type suitable for the present invention is, for example, polydimethylsiloxane and cyclomethylsiloxane, and also aryl- or alkyl- or alkoxy-substituted polymethylsiloxane and cyclomethylsiloxane.

The oil/fat suitable for the present invention also comprises mono- or diesters of linear and/or branched mono- and/or dicarboxylic acids having 2 to 44 carbon atoms with saturated or unsaturated, linear and/or branched alcohols having 1 to 22 carbon atoms. Likewise, as the oil/fat used in the present invention, use can also be made of the esters of bifunctional aliphatic alcohols having 2-36 carbon atoms with monofunctional aliphatic carboxylic acids having 1-22 carbon atoms.

As the oil/fat used in the present invention, use can also be made particularly of esters of fatty acids having 12-22 carbon atoms, such as methyl esters and isopropyl esters, such as methyl laurate, methyl stearate, methyl oleate, methyl erucate, isopropyl palmitate, isopropyl myristate, isopropyl stearate and/or isopropyl oleate.

Furthermore, as the oil/fat used in the present invention, preference is also given particularly to n-butyl stearate, n-hexyl laurate, n-decyl oleate, isooctyl stearate, isononyl palmitate, isononyl isononanoate, 2-ethylhexyl palmitate, 2-ethylhexyl laurate, 2 hexyl-decyl stearate, 2-octyldodecyl palmitate, oleyl oleate, oleyl erucate and/or erucyl oleate.

As the oil/fat used in the present invention, dicarboxylic esters are also especially suitable, such as di(n-butyl) adipate, di(n-butyl) sebacate, di(2-ethylhexyl) adipate, di(2-hexyldecyl) succinate and/or diiso-tridecyl azelate. As the oil/fat used in the present invention, diol esters are also especially suitable, such as ethylene glycol dioleate, ethylene glycol diisotri-decanoate, propylene glycol di(2-ethylhexanoate), butanediol diisostearate and/or neopentyl glycol dicaprylate.

As the oil/fat used in the present invention, use can also be made of carbonic acid diesters, such as diethylhexyl carbonate.

Relatively long-chained triglycerides are also suitable for use, i.e., triple esters of glycerol with three acid molecules, at least one of which is a relatively long-chain acid molecule. Mention can be made here, by way of example, of fatty acid triglycerides, which comprise synthetic triglycerides of caprylic/capric acid mixtures, triglycerides of industrial oleic acid, triglycerides of isostearic acid and triglycerides of palmitic/oleic acid mixtures. Use can additionally be made of linear or branched fatty alcohols, such as oleyl alcohol or octyldodecanol, and also fatty alcohol ethers, such as dioctyl ether, PPG-3 myristyl ether, etc.

As the oil/fat used in the present invention, natural vegetable oils are also suitable for use, for example, olive oil, sunflower oil, soybean oil, peanut oil, rapeseed oil, almond oil, palm oil or jojoba oil, and also the liquid portions of coconut oil or palm kernel oil, and also the liquid portions of animal fats, such as sperm oil, neatsfoot oil or beef tallow.

As the oil/fat used in the present invention, use can also be made of hydrocarbon oils/fats, particularly liquid paraffins and isoparaffins. Examples of hydrocarbon oils/fats which can be used are paraffin oils, white mineral oils, isohexadecane, polydecene, petroleum jelly, light liquid paraffins or squalane. Furthermore, esters of arylcarboxylic acids are also suitable, such as, esters of benzoic acid, e.g., benzoic acid esters formed by the esterification of saturated or unsaturated, linear or branched alcohols having 1 to 22 carbon atoms with benzoic acid, such as isostearyl benzoate and octyldodecyl benzoate, preferably C₁₂₋₁₅-alkyl benzoates.

In a preferred embodiment of the present invention, the components of the oil phase are preferably selected from one or more of the following groups: di-ethylhexyl carbonate, cyclomethylsiloxane, cetyl polydimethylsiloxane and polydimethylsiloxane.

In the water-in-oil emulsion system of the present invention, the water phase forms the disperse phase of the water-in-oil emulsion system. In the water phase, it can only comprise water, and apart from water it can also comprise other substances soluble in water. These substances can be those normally contained in the water phase in terms of the oil-in-water emulsion of the preparations for personal care and for makeup. Especially this includes ethanol and/or C2-C5 polyols having two or more (for example three) hydroxyl groups, and the latter is preferably glycerol, propylene glycol, 1,3-butanediol or any mixture thereof, and especially glycerol. In addition, the water phase can also comprise a water soluble polymer, which is selected from one or more of the following groups: xanthan gum, guar gum, chondroitin sodium sulphate, sodium hyaluronate, arabic gum, sodium alginate, carrageenan, hydroxypropyl cellulose, methylcellulose, substituted methylcellulose (such as hydroxypropylmethyl cellulose), polyacrylic acid, and polyacrylic acid having alkyl substituents on the main chain (for example, TEGO^{®} Carbomer 341 ER). The water soluble polymer is especially xanthan gum.

Of course, those skilled in the art should understand that, in addition to the above mentioned components, the water-in-oil emulsion system of the present invention can also comprise other auxiliary components, such as humectant, emollient, free radical scavenger, chelator, antioxidant, essence, preservative (for example 2-brom-2-nitro-1,3-propylene glycol), film-forming agent, stabilizer (such as sodium chloride, magnesium chloride and magnesium sulfate), etc. These auxiliary components are included in the oil phase or the water phase according to their solubilities in the water phase and in the oil phase.

In the water-in-oil emulsion system of the present invention, any liposoluble or lipo-dispersible component normally used in cosmetics, such as sunscreening agent and hydrophobic modified powder, can also be added therein.

The sunscreening agent, such as a UV sunscreening agent, can be for example organic substances, which absorb ultraviolet radiation and radiate out the absorbed energy by a longer wavelength, such as heat.

The UV-B sunscreening agent can be oil soluble or water soluble; if an oil soluble UV-B sunscreening agent is used, it will be contained in the oil phase of the emulsion of the present invention; and if a water soluble UV-B sunscreening agent is applied, it will be contained in the water phase of the emulsion of the present invention.

As the oil soluble UV-B sunscreening agent, mention can be made of, for example:
3-benzylidenecamphor and the derivatives thereof, for example, 3-(4-methyl benzylidene)camphor;
4-aminobenzoic acid derivatives, for example, 2-ethylhexyl 4-(dimethylamino)benzoate and pentyl 4-(dimethylamino)benzoate;
cinnamates, for example, 2-ethylhexyl 4-methoxy-cinnamate, isopentyl 4-methoxy-cinnamate and 2-ethylhexyl 2-cyano-2-phenylcinnamate (Octocrilene);
salicylates, for example, 2-ethylhexyl salicylate, 4-isopropylbenzyl salicylate and sym-menthyl salicylate;
benzophenone derivatives, for example, 2-hydroxyl-4-methoxy benzophenone, 2-hydroxyl-4-methoxy-4'-methyl benzophenone and 2,2'-dihydroxyl-4-methoxy benzophenone;
benzylidene malonates, for example, di(2-ethylhexyl) 4-methoxy-benzylidene-malonate;
triazine derivatives, for example, 2,4,6-trianilino-(p-carbon-2'-ethyl-1'-hexoxy)-1,3,5-triazine and octyl triazone; and
propyl-1,3-diketones, for example, 1-(4-tert-butyl phenyl)-3-(4'-methoxy phenyl) propyl-1,3-diketone.

The water soluble UV-B sunscreening agents that can be used are:
2-phenylbenzimidazole-5-sulfonic acid and alkali metals, alkaline earth metals, ammonium, alkyl ammonium, alkanol ammonium and glucammonium salts thereof;
derivatives of benzophenone sulfonic acid and the salts thereof, for example, 2-hydroxyl-4-methoxy benzophenone-5-sulfonic acid and the salts thereof; and
sulfonic derivatives of 3-benzylidenecamphor and the salts thereof, for example, 4-(2-oxy-3-bornylidene-methyl) benzenesulfonic acid, 2-methyl-5-(2-oxy-3-bornylidene) benzenesulfonic acid and the salts thereof.

Derivatives of benzoylmethane can particularly be used as typical UV-A sunscreening agents, for example, 1-(4'-(tert-butyl)phenyl)-3-(4'-methoxyl phenyl)propyl-1,3-diketone and 1-phenyl-3-(4'-isopropyl phenyl)propyl-1,3-diketone. Apparently, UV-A and UV-B sunscreening agents can also be used in the form of a mixture.

Furthermore, other suitable UV sunscreening agents can be found in the review of SÖFW-magzine by P. Finkel, volume 122, p. 543 (1996).

In addition to the abovementioned two main groups of UV sunscreening agents, use can also be made of secondary sunscreening agents of antioxidant type, which break the photochemical reaction chain; if UV radiation penetrates the skin, the reaction chain will be triggered. As the antioxidant, use can be made of, for example, superoxide dismutase, tocopherol (vitamin E), 2,6-dihydroxyl butyl toluene and ascorbic acid (vitamin C).

As insectifuge, use can be made of, for example, N,N-diethyl-m-toluamide, 1,2-pentadiol and/or insectifuge 3535.

As suntanning agent, use can be made of, for example, dihydroxyacetone and erythrulose.

As preservative, use can be made of, for example, a mixture of one or more of alkyl p-hydroxybenzoates and phenoxyethanol. The alkyl p-hydroxybenzoates can be methyl p-hydroxybenzoate, ethyl p-hydroxybenzoate, propyl p-hydroxybenzoate and/or butyl p-hydroxybenzoate. Other alcohols can be used to replace phenoxyethanol, for example, benzyl alcohol or ethanol. Additionally, other common preservatives can also be used, for example, sorbic acid, benzoic acid, salicylic acid, 2-brom-2-nitropropane-1,3-diol, chloroacetamide, diazolidinyl urea, DMDM glycolyurea, sodium hydroxymethylglycinate, methyl isothiazoline, chloromethyl isothiazoline, ethylhexyl glycerol and/or capryloyl ethanediol.

As spice, use can be made of natural or synthetic scented substances or mixtures thereof. Natural scented substances comprise extracts of flowers (lily, lavender, rose, jasmine, neroli, ylang-ylang), stems and leaves (geranium, patchouli, petitgrain), fruits (aniseed, coriandrum sativum, caraway, juniper), peels (bergamot orange, lemon, orange), roots (muscade, angelica, celery, cardamom, saussureaeradix, fleur-de-lis, thyme), needle leaves and stems (spruce, abies delavayi, pine, mountain pine) and resins and balsams (galbanum, elemi, benzoin, myrrh, frankincense, opopanax). Use can also be made of scent materials of animal origin, for example, from civet and castoreum. Typical synthetic spice compounds are ester, ether, aldehyde, ketone, alcohol and hydrocarbon type products. The ester-type spice compounds are, for example, benzylacetate, phenoxyethyl isobutyrate, p-tert-butylcyclohexyl acetate, linalyl acetate, phenylethyl acetate, linalyl benzoate, benzyl formate, phenylglycine methyl ester, allyl cyclohexylpropionate, styrallyl propionate and benzyl salicylate; the ether-type spice compounds comprise, for example, benzyl ethyl ether; the aldehyde-type spice compounds comprise, for example, linear alkyl aldehydes having 8-18 carbon atoms, citral, citronellal, citronellyl oxyacetaldehyde, cyclamenaldehyde, hydroxycitronellal, lilial and bourgeonal; the ketone-type spice compounds comprise, for example, ionones, isomethyl ionone and methyl cedryl ketone; the alcohol-type spice compounds comprise anisole, citronellol, eugenol, isoeugenol, geraniol, linalool, phenethanol and terpineol; and the hydrocarbon-type spice compounds mainly comprise terpenes and balsams. Use can be made of mixtures of various scented substances, which together generate an attractive scent. Low volatile oils are generally used as flavor components, and are also suitable as spice, for example, sage oil, chamomile oil, clove oil, balsam oil, peppermint oil, cinnamon leaves oil, oil of tiliae flos, oil of juniper seed, vetiver oil, olibanum oil, galbanum oil, cistus oil and lavandin oil. The following can be used alone or in a mixture: bergamot oil, dihydromyrcenol, lilial, lyral, citronellol, phenethanol, α-hexyl-cinnamic aldehyde, geraniol, benzylacetone, cyclamen aldehyde, linalool, boisambrene forte, ambroxan, indole, Hedione, lemon oil, mandarin oil, tangerine oil, allyl amyl glycolate, lavandin oil, sage oil, damascone, geranium oil, cyclohexyl salicylate, methyl cedryl ketone, phenylacetic acid, geranyl acetate, benzylacetate and rose oxide.

As colorant, substances suitable for use and approved to be used in cosmetics are those listed in the publication Kosmetische Färbemittel[colorants for cosmetics], Farbstoffkommission der Deutschen Forschungsgemeinschaft [Colorant Commission of the German Research Association], Verlag Chemie, Weinheim, 1984, p. 81-106. On the basis of the total emulsion mixture, these colorants are used only in a concentration of 0.001-0.1 % by weight.

In the water-in-oil emulsion system of the present invention, conventional amounts of active substances from biological sources can also be added. The term "active substances from biological sources" is to be understood as referring to, for example, tocopherol and derivatives thereof, ascorbic acid and derivatives thereof, retinol and derivatives thereof, deoxyribonucleotide, coenzyme Q10, bisabolol, allantoin, phytantriol, panthenol, hydroxy acids, salicylic acid, amino acids and derivatives thereof, hyaluronic acid, glucan, creatine and derivatives thereof, guanidine and derivatives thereof, ceramide, phytosphingosine and derivatives thereof, sphingosine and derivatives thereof, pseudo-ceramide, volatile oils, peptides, protein hydrolysate, plant extracts, vitamins and mixtures of vitamins.

In the water-in-oil emulsion system of the present invention, water-in-oil emulsifiers are used, which are those commonly used in water-in-oil emulsions. The emulsifier is preferably a surfactant with an HLB value of 2-8, preferably at least one selected from the following three types (in which the hydrophilic group is a polyether glycol group, and preferably polyoxyethylene, polyoxypropylene, polyglyceryl or polyoxyethylene sorbitan):
the first category: an emulsifier having a molecular chain structure of polysiloxane + a hydrophilic group + an alkyl group, which comprises a block copolymer emulsifier with polysiloxane, a polyetherpolyol and a C₁-C₂₂ aliphatic alkane being connected by covalent bonds, an emulsifier with polysiloxane as a main chain, and polyetherpolyol and a C₁-C₂₂ aliphatic alkane as side groups being respectively connected to the polysiloxane chain by covalent bonds, and an emulsifier with polysiloxane as a main chain, C₁-C₂₂ aliphatic alkyl modified polyetherpolyol as a side group being connected to the polysiloxane chain by covalent bonds; it is preferably C₆-C₂₀ alkyl copolymerized polyoxyethelene polydimethylsiloxane of the following formula, it is more preferably cetyl PEG/PPG-10/1 polydimethylsiloxane (provided by Evonik Degussa with the trade name of ABIL^{®} EM90): 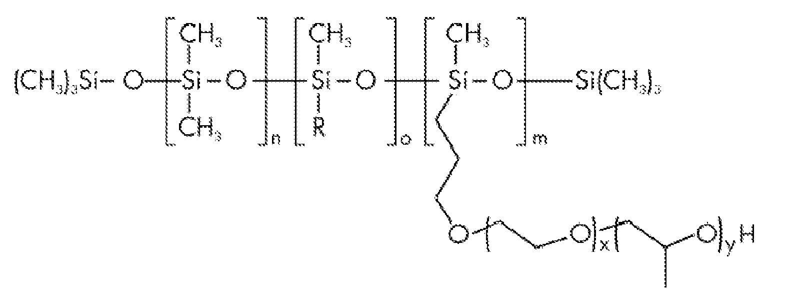 wherein R is a C₆-C₂₀ alkyl group, x=10, y=1, n=1-200, 0=1-100, and m=1-40;
the second category: an emulsifier having a molecular chain structure of polysiloxane + a hydrophilic group, which emulsifier comprises a block copolymer emulsifier with polysiloxane and a polyetherpolyol being connected by covalent bonds, an emulsifier with polysiloxane as a main chain and polyetherpolyol as a side group which is connected to the main chain of polysiloxane by covalent bonds; it is preferably polyoxyethylene polydimethylsiloxane, polyglycerol polydimethylsiloxane; it is more preferably bis-PEG/PPG-14/14 polydimethylsiloxane (provided by Evonik Degussa with the trade name of ABIL^{®} EM97); and
the third category: an ester-type emulsifier formed by polyether glycol or polyol of nonlinear structure or linear structure with its hydroxy group and C₁-C₂₂ fatty acid, or an ether-type emulsifier formed by polyether glycol or polyol with its hydroxy group and C₁-C₂₂ aliphatic alcohol, which emulsifier comprises polyoxyethylene fatty acid ester, polyoxyethylene alkyl ether, mono-or polyglycerol ester of fatty acids, mono- or polyglycerol alkyl ether, preferably di-isostearyl polyglyceryl-3 dipolylinoleate (provided by Evonik Degussa with the trade name of ISOLAN^{®} PDI), polyglyceryl-4 diisostearate /polyhydroxystearate/sebacate (provided by Evonik Degussa with the trade name of ISOLAN^{®} GPS), polyoxyethylene sorbitan fatty acid ester.

The formulation of the water-in-oil emulsion system of the present invention contains solid powder with the surface prefereably being hydrophobically treated or liquid powder paste formulated by the solid powder and oil/fat and an emulsifier. The solid powder comprises nano-scale powder, micron-scale powder and a mixture of these two in any proportion, with the mean diameter of the micron-scale powder being between 500nm-5µm and the primary particle diameter of the nano-scale powder between 5-50nm. The powder used in the present invention comprises inorganic oxide particles, pigment particles and polymer particles usually used in the personal care field. Examples that can be mentioned are titanium dioxide, zinc oxide, silicon dioxide, chromium oxide, iron blue, and carbon black, barium, strontium, calcium and aluminum precipitation lakes, talc, smectite, kaolin, mica, Nylon^{®} powder (for example Orgasol^{®} of Atochem company), polyethylene powder, Teflon^{®}, and silicone resin microbead (for example Tospearl^{®} of Toshiba company).

The cationic surfactants suitable for the present invention comprise the cationic surfactants of quaternary ammonium salts, represented by the general formula R₁R₂R₃R₄N⁺X⁻, wherein R₁, R₂, R₃ and R₄ are each independently selected from a C₁-C₂₈ alkyl group or a C₁-C₂₈ alkyl group substituted by a C₁-C₄ acyl group, a C₂-C₄ acyloxy group, a hydroxy group or a C₂-C₄ alkenyl group, preferably a C₁₂-C₂₂ alkyl group or a C₁₂-C₂₂ alkyl group substituted by a C₁-C₄ acyl group, a C₂-C₄ acyloxy group, a hydroxy group or a C₂-C₄ alkenyl group, X⁻ is any suitable anion, such as Cl⁻, Br or CH₃SO₃⁻ ; more preferably three of groups R₁-R₄ are methyl groups; more preferably being selected from palmitoylpropyl trimethylammonium chloride (VARISOFT^{®} PATC, EVONIK), hexadecyltrimethylammonium chloride, tetradecyltrimethylammonium chloride, docosyltrimethylammonium chloride, docosyltrimethylammonium methylsulfate or a mixture thereof.

The molecular weight of the cationic surfactant involved in the present invention is preferably below 600g/mol.

In the water-in-oil emulsion system of the present invention, the amount of the solid powder is preferably 1-20 wt% on the basis of the total amount of the system; the amount of the cationic surfactant is preferably 0.1-1 wt% on the basis of the total amount of the system.

Said water-in-oil emulsion system with low viscosity in the present invention refers to a water-in-oil emulsion system with a viscosity lower than or equal to 5000mPa.s measured at 25°C using a Brookfield viscometer and a rotor SP62 at 30rpm.

The stability of the water-in-oil emulsion system of the present invention was generally evaluated by means of centrifugation observation. Particularly, the water-in-oil emulsion system of the present invention was continuously centrifuged for 30min at 3000rpm; if no phenomena of demulsification, delamination, phase separation or oil-water separation are observed, the system is considered to be stable.

The cationic surfactant has the function of lowering viscosity while improving emulsion stability of the powder-containing water-in-oil emulsion system. However, the cationic surfactant also has a function of lowering viscosity of a common water-in-oil emulsion system, while having no apparent function of improving the stability.

### Particular Embodiments

The following embodiments and comparative examples are used to further describe the present invention by comparison, but they in no way limit the scope of the present invention.

Unless otherwise specified, the percentage refered to in the present invention is the mass percentage.

### Comparative examples 1-2 and embodiments 1-3

Emulsions were prepared according to the formulations in Table 1 below. The particular preparation procedure was as follows:
1) a titanium dioxide paste (if used) was dispersed through high-speed stirring in the oil/fat and the emulsifier of phase A to prepare phase A,
2) water, glycerol, VARISOFT^{®} PATC (if used), sodium chloride and a preservative were mixed homogeneously to prepare phase B,
3) the phase B was added slowly under stirring into phase A, with the system being maintained in a homogeneously dispersed state during the stirring, and
4) the same was stirred and homogenized for 3min at 1300rpm.

**Table 1**

| | | Comparative example 1 | Comparative example 2 | Embodiment 1 | Embodiment 2 | Embodiment 3 |
|---|---|---|---|---|---|---|
| A | Cetyl PEG/PPG-10/lpolydimethylsiloxane ABIL^{®} EM 90 | 2.0 | 2.0 | 2.0 | 2.0 | 2.0 |
| | Diethylhexyl carbonate TEGOSOFT^{®}DEC | 7.0 | 19.0 | 7.0 | 7.0 | 9.0 |
| | Cyclomethylsiloxane DC 345 | 17.0 | 4.0 | 17.0 | 17.0 | 14.0 |
| | Titanium dioxide paste TEGO^{®}SUN TDEC 45 | 14.0 | 0 | 14.0 | 14.0 | 10 |
| B | Water | 54 | 68.94 | 53.7 | 53.4 | 58.85 |
| | Glycerol | 5.0 | 5 | 5.0 | 5.0 | 5.0 |
| | Palmitoylpropyl trimethylammonium chloride VARISOFT^{®} PATC | 0 | 0.06 | 0.3 | 0.6 | 0.15 |
| | NaCl | 1.0 | 1.0 | 1.0 | 1.0 | 1.0 |
| | Preservative | Quantum sufficit | Quantum sufficit | Quantum sufficit | Quantum sufficit | Quantum sufficit |
| Viscosity(sp62 30rpm) mPa.s | | 1184 | 149 | 980 | 437.3 | 714.7 |
| | | | | | | |
| Centrifugation result (3000rpm , 30min) | | Phase separation | Phase separation | Pass | Pass | Pass |
| Thermostability 45°,1 month | | Phase separation | Phase separation | Pass | Pass | Pass |

| | | | | | | |
|---|---|---|---|---|---|---|
| Note: Titanium dioxide was provided by Evonik company in the form of TEGO^{®}SUN TDEC 45 paste, which contained 45 wt% titanium dioxide with a mean diameter of about 100nm and the surface being hydrophobically treated, and the balance were oil/fat and surfactants. | | | | | | |

Palmitoylpropyl trimethylammonium chloride was provided by Evonik company with the trade name VARISOFT^{®} PATC, in which the content of the active substances was 60 wt% and the balance was isopropanol.

In the present application, reagents under the trade names of TEGOSOFT^{®}, ABIL^{®}, TEGO^{®}, ISOLAN^{®}, AEROSIL^{®} and AMILAN^{®} were all provided by EVONIK company; and DC 345 was provided by Dow-Corning company.

The preservatives used in the present application were mixtures of nipagin and phenoxyethanol, provided by Schulz company in Germany with the trade name K145.

The viscosities of emulsions 1-3 obtained from embodiments 1-3 were respectively: 980mPa.s, 437.3mPa.s and 714.7mPa.s (measured at sp62, 30rpm). By centrifugation stability tests, there were no phenomena of delamination and demulsification when centrifuged for 30min at a speed of 3000rpm. Emulsions 1-3 were also subjected to a thermostability test, by keeping it standing at 45°C for 1 month, and no phenomena of delamination and demulsification were observed. The above indicated that the emulsions prepared in embodiments 1-3 had good stability.

On the basis of embodiment 1, 0.3 wt% of palmitoylpropyl trimethylammonium chloride was replaced by corresponding amounts of TEGIN PL Flake anion emulsifier, ABIL^{®} Care XL 80 nonionic emulsifier and REWOPOL SB Z anion emulsifier provided by Evonik company, so as to study the influences of various ionic hydrophilic emulsifiers on the centrifugation stability of the W/O powder-containing system. As a result, it was found that oil-water separation phenomenon was observed in all of these comparative products, which indicated that other various ionic hydrophilic emulsifiers had no effect on improving the stability of the powder-containing emulsion system.

Embodiment 3 indicated that the amount of palmitoylpropyl trimethylammonium chloride could be reduced correspondingly when the amount of the powder was reduced.

### Comparative examples 3-5 and embodiments 4-6

Emulsions were prepared according to the formulations in Table 2 below. The particular preparation procedure was as follows:
1) TiO₂ powder or ZnO powder or SiO₂ powder was dispersed through high-speed stirring in the oil/fat and the emulsifier of phase A to prepare phase A,
2) water, glycerol, VARISOFT^{®} PATC (if used), sodium chloride and a preservative were mixed homogeneously to prepare phase B,
3) the phase B was added slowly under stirring into phase A, with the system being maintained in a homogeneously dispersed state during the stirring, and
4) the same was stirred and homogenized for 3min at 1300rpm.

**Table 2**

| | | Comparative example 3 | Embodiment 4 | Comparative example 4 | Embodiment 5 | Comparative example 5 | Embodiment 6 |
|---|---|---|---|---|---|---|---|
| A | ABIL^{®} EM 90 | 2.0 | 2.0 | 2.0 | 2.0 | 2.0 | 2.0 |
| | TEGOSOFT^{®} DEC | 8.7 | 6.4 | 8.7 | 8.7 | 10.0 | 10.0 |
| | DC 345 | 8.0 | 6.5 | 8.0 | 8.0 | 10.0 | 10.0 |
| | TiO₂ powder (MT100-TV, Tayca) | 6.3 | 6.3 | 0 | 0 | 0 | 0 |
| | ZnO powder TEGO^{®}SUN Z 800 | 0 | 0 | 6.3 | 6.0 | 0 | 0 |
| | SiO₂ powder AEROSIL R 812 S | 0 | 0 | 0 | 0 | 2.0 | 2.0 |
| B | Water | 69.0 | 72.5 | 69.0 | 69.0 | 70 | 69.7 |
| | Glycerol | 5.0 | 5.0 | 5.0 | 5.0 | 5 | 5 |
| | VARISOFT^{®} PATC | 0 | 0.3 | 0 | 0.3 | 0 | 0.3 |
| | NaCl | 1.0 | 1.0 | 1.0 | 1.0 | 1.0 | 1.0 |
| | Preservative | Quantum sufficit | Quantum sufficit | Quantum sufficit | Quantum sufficit | Quantum sufficit | Quantum sufficit |
| Viscosity (sp62, 30rpm) mPa.s | | 4682 | 4160 | 6144 | 2410 | 27360 | 2784 |
| Centrifugation result (3000rpm. 30min) | | High yield of oiL with a small amount of powder agglomerates | Pass | High yield of oiL powder agglomerates | Pass | High yield of oil | Pass |

### Comparative examples 6-8 and embodiment 7

Emulsions were prepared according to the formulations in Table 3 below. The particular preparation procedure was as follows:
1) a titanium dioxide paste was dispersed through high-speed stirring in the oil/fat and the emulsifier of phase A to prepare phase A,
2) water, glycerol, VARISOFT^{®} PATC (if used) or quaternary ammonium salt-80 (if used) or guar gum hydroxypropyl trimethylammonium chloride AMILAN^{®} (if used), sodium chloride and a preservative were mixed homogeneously to prepare phase B,
3) the phase B was added slowly under stirring into phase A, with the system being maintained in a homogeneously dispersed state during the stirring, and
4) the same was stirred and homogenized for 3min at 1300rpm.

**Table 3**

| | | Comparative example 6 | Embodiment 7 | Comparative example 7 | Comparative example 8 |
|---|---|---|---|---|---|
| A | Polyglyceryl-4 diisostearate/ polyhydroxystearate/sebacate ISOLAN^{®} GPS | 2.0 | 2.0 | 2.0 | 2.0 |
| | Diethylhexyl carbonate TEGOSOFT^{®}DEC | 8.0 | 8.0 | 8.0 | 8.0 |
| | Cyclomethylsiloxane DC 345 | 11.0 | 11.0 | 11.0 | 11.0 |
| | Titanium dioxide paste TEGO^{®}SUN TDEC 45 | 14.0 | 14.0 | 14.0 | 14.0 |
| B | Water | 59 | 59 | 59 | 59 |
| | Glycerol | 5.0 | 4.7 | 5.0 | 4.7 |
| | Palmitoylpropyl trimethylammonium chloride VARISOFT^{®} PATC | 0 | 0.3 | 0 | 0 |
| | Quaternary ammonium salt-80 ABIL^{®} QUAT 3237 | 0 | 0 | 0.3 | 0 |
| | Guar gum hydroxypropyl trimethyl ammonium chloride AMILAN^{®} GUAR-36 | 0 | 0 | 0 | 0.3 |
| | NaCl | 1.0 | 1.0 | 1.0 | 1.0 |
| | Preservative | Quantum sufficit | Quantum suflicit | Quantum sufficit | Quantum suflicit |
| Viscosity (sp62, 30rpm) mPa.s | | 1877 | 1173 | | |
| Centrifugation result (3000rpm, 30min) | | Phase separation | Pass | Phase separation | Phase separation |

Embodiment 7, which used polyglyceryl-4diisostearate/polyhydroxystearate/sebacate ISOLAN^{®} GPS as the emulsifier, can still arrive at a product with suitably lowered viscosity and good stability.

Equivalent cationic polymer quaternary ammonium salt-80 (ABIL^{®} QUAT 3237 Quaternium-80) of high molecular weight or guar gum hydroxypropyl trimethylammonium chloride ( AMILAN^{®} GUAR-36 ) was used to replace the palmitoylpropyl trimethylammonium chloride in embodiment 7, the comparative products 7 and 8 obtained were very unstable, which all had the phenomena of phase separation and powder agglomeration after the centrifugation test for 30min at 3000rpm.

### Embodiment 8

Sunblock was prepared according to the formulations in Table 4 below. The particular preparation procedure was as follows:
1) the three types of powder or powder paste, namely a nano-scale titanium dioxide powder paste (TEGO^{®} SUN TDEC 45), micron-scale talc powder and nano-scale zinc oxide powder (TEGO^{®}SUN Z 800), were dispersed through high-speed stirring in the oil/fat and the emulsifier of phase A to prepare phase A,
2) water, glycerol, VARISOFT^{®} PATC, triethanolamine and a preservative were mixed, then two water soluble sunscreening agents, namely 2-phenylbenimidazole-5-sulfonic acid and disodium phenyl-bisbenzimidazole-tetra-disulfate, were dissolved in the obtained mixture to obtain phase B,
3) the phase B was added slowly under stirring into phase A, with the system being maintained in a homogeneously dispersed state during the stirring, and
4) the same was stirred and homogenized for 3min at 1300rpm.

**Table 4 Sunblock**

| | | wt% |
|---|---|---|
| A | ABIL^{®} EM 90 | 2.0 |
| | TEGOSOFT^{®} DEC | 4.4 |
| | Cetyl polydimethylsiloxane ABIL^{®} WAX 9801 | 1.0 |
| | DC 345 | 16.0 |
| | Polydimethylsiloxane ABIL^{®} 350 | 4.0 |
| | Octyl methoxycinnamate | 7.0 |
| | Titanium dioxide paste TEGO^{®} SUN TDEC 45 | 15.6 |
| | Talcum powder | 2.0 |
| | TEGO^{®}SUN Z 800 | 2.0 |
| B | Water | 35.94 |
| | Glycerol | 5.0 |
| | VARISOFT^{®} PATC | 0.3 |
| | 2-phenylbenimidazole-5-sulfonic acid | 2.0 |
| | Disodium phenyl-bisbenzimidazole-tetra-disulfate | 1.0 |
| | Triethanolamine | 1.76 |
| | Preservative | Quantum sufficit |

The viscosity of the sunblock obtained was 900mPa.s (measured at sp62, 30rpm). After the centrifugation stability test, no phenomena of delamination and demulsification were observed when centrifuged for 30min at a speed of 3000rpm. The sunblock was also subjected to a thermostability test by keeping it standing for 1 month at 45°C, and no phenomena of delamination and demulsification were observed. This indicated that the products prepared in this embodiment had good stability.

### Embodiment 9

The foundation was prepared according to the formulations in Table 5 below. The particular preparation procedure was as follows:

**Table 5 Foundation**

| | | wt% |
|---|---|---|
| A | ABIL^{®} EM 90 | 2.5 |
| | Copolymer of cyclomethylsiloxane and dimethyl silicone DC 9040 | 10.0 |
| | Bentonite gel | 5.0 |
| | DC 345 | 14.2 |
| B | Iron oxide, micron-scale powder | 0.72 |
| | TiO₂ (AS micron-scale powder) | 7.2 |
| | TEGOSOFT^{®} DEC | 10.0 |
| C | Water | 33.38 |
| | Glycerol | 15.0 |
| | VARISOFT^{®} PATC | 1.0 |
| | NaCl | 1.0 |
| | Preservative | Quantum sufficit |

1) iron oxide toner powder of micron-scale and titanium dioxide powder of micron-scale were dispersed through high-speed stirring in the oil/fat diethylhexyl carbonate (TEGOSOFT^{®} DEC) to prepare phase B,
2) the components of phase A were mixed to prepare phase A, and the phase A was mixed with phase B homogeneously,
3) the components of phase C were mixed to prepare phase C, and the phase C was added slowly under stirring at a steady speed into the mixture of phase A and phase B prepared in step 2, and
4) the same was stirred and homogenized for 3min at 1300rpm.

The viscosity of the foundation obtained was 853mPa.s (measured at sp62, 30rpm). After the centrifugation stability test, no phenomena of delamination and demulsification were observed when centrifuged for 30min at a speed of 3000rpm. The foundation was also subjected to a thermostability test by keeping it standing for 1 month at 45°C, and no phenomena of delamination and demulsification were observed. This indicated that the products prepared in this embodiment had good stability.

## Claims

1. A water-in-oil emulsion system, comprising, on the basis of the total amount of the system,
30-90 wt% of a water phase,
5-65 wt% of an oil phase,
1-5 wt% of a water-in-oil emulsifier,
0.1-20 wt% of a solid powder and
0.01-2 wt% of a cationic surfactant,
wherein the cationic surfactant is the cationic surfactant of a quaternary ammonium salt represented by the general formula R₁ R₂R₃R₄N⁺X⁻, in which
R₁, R₂, R₃ and R₄ are each independently selected from a C₁-C₂₈ alkyl group or a C₁-C₂₈ alkyl group substituted by a C₁-C₄ acyl group, a C₂-C₄ acyloxy group, a hydroxy group or a C₂-C₄ alkenyl group, and
X- is any suitable anion.

2. The water-in-oil emulsion system as claimed in claim 1, comprising, on the basis of the total amount of the system,
45-85 wt% of a water phase,
10-45 wt% of an oil phase,
1-5 wt% of an emulsifier,
1-20 wt% of a solid powder and
0.01-2 wt% of a cationic surfactant of the quaternary ammonium salt, with the quaternary ammonium salt represented by the general formula R₁R₂R₃R₄N⁺X⁻, in which at least one of R₁, R₂, R₃ and R₄ is selected from a C₁₂-C₂₂ alkyl group or a C₁₂-C₂₂ alkyl group substituted by a C₁-C₄ acyl group, a C₂-C₄ acyloxy group, a hydroxy group or a C₂-C₄ alkenyl group, and X⁻ is Cl⁻, Br or CH₃SO₃-.

3. The water-in-oil emulsion system as claimed in claim 1 or 2, wherein three of groups R₁-R₄ in the cationic surfactant of the quaternary ammonium salt are methyl groups.

4. The water-in-oil emulsion system as claimed in claims 1-3, wherein the molecular weight of the cationic surfactant of the quaternary ammonium salt is below 600g/mol.

5. The water-in-oil emulsion system as claimed in claim 4, wherein the cationic surfactant of the quaternary ammonium salt is selected from palmitoylpropyl trimethylammonium chloride, tetradecyltrimethylammonium chloride, hexadecyltrimethylammonium chloride, docosyltrimethylammonium chloride, docosyltrimethylammonium methylsulfate or a mixture thereof.

6. The water-in-oil emulsion system as claimed in claim 4 or 5, wherein, on the basis of the total amount of the system, the amount of the cationic surfactant of the quaternary ammonium salt is between 0.1-1wt%.

7. The water-in-oil emulsion system as claimed in claims 1-3, wherein the viscosity of the water-in-oil emulsion system is lower than or equal to 5000 mPa.s measured at 25°C using a Brookfield viscometer and a rotor SP62 at 30 rpm.

8. The water-in-oil emulsion system as claimed in claims 1-7, wherein the powder is selected from a nano-scale powder, a micron-scale powder and a mixture of these two by any proportion.

9. Use of a cationic surfactant in improving the stability of a water-in-oil emulsion system.
